# EUROPÄISCHE PATENTSCHRIFT

(11) **EP 1 903 102 B1**
(45) Veröffentlichungstag und Bekanntmachung des Hinweises auf die Patenterteilung: **13.07.2011**
(21) Anmeldenummer: 07015847.2
(22) Anmeldetag: 11.08.2007
(51) Int. Cl.: C12M 1/107, B02C 13/00, B02C 18/14

(54) **Vorrichtung zur Förderung von Biomassematerialien**
Device for conveying biomass materials
Dispositif destiné au transport de matériaux de biomasse

(30) Priorität: 25.09.2006 DE 102006045537; 25.09.2006 DE 202006017085 U; 10.11.2006 DE 102006053336
(43) Veröffentlichungstag der Anmeldung: 26.03.2008
(73) Patentinhaber: Huning Maschinenbau GmbH, 49324 Melle (DE)
(72) Erfinder: Huning-Wesseler, Walter, 49326 Melle (DE)
(74) Vertreter: Pott, Ulrich

(56) Entgegenhaltungen:
- DE-A1- 10 335 881
- DE-C1- 19 617 734
- DE-U1-202004 012 014

## Beschreibung

Die Erfindung bezieht sich auf eine Vorrichtung zur Förderung von Biomassematerialien in z.B. einen Fermenter mit zumindest einer beispielsweise eine Förderschnecke als Förderer aufweisenden Förderstufe und mit einem der Förderstufe zugeordneten, Zerkleinerungswerkzeuge aufweisenden Zerkleinerer, beispielsweise einer Mühle.

Anlagen zur Förderung von Biomassematerialien sind bekannt und haben beispielsweise eine Vertikalförderschnecke als Vertikalförderstufe, der ein Aufgabeband vorgeordnet und eine Zerkleinerungsmühle nachgeordnet ist, gegebenenfalls auch mit sich daran anschließenden Förderschnecken zwecks Überführung in einzelne Fermentereinheiten, wobei die Zerkleinerungsvorrichtung für eine Vorbehandlung der Biomassematerialien Sorge zu tragen hat. Bei bekannten Vorrichtungen wird die Zerkleinerung der Biomassematerialien von der Mühle vorgenommen, wobei die verwendeten Zerkleinerungswerkzeuge das Biomassematerial unabhängig von dessen Konsistenz zerkleinern. Dabei werden in aller Regel alle aufgegebenen Biomassematerialien durch die Mühle zugeführt, was dazu führt, daß der Förderkapazität Grenzen gesetzt sind. Ebenfalls ist es nicht möglich, in Abhängigkeit der aufzugebenen Biomassematerialien ein optimal vorbehandeltes Produkt für einen optimierten Gärprozeß einem Fermenter zuzuführen. Das beeinträchtigt nachproduzierbare und mithin industriell planbare Gärprozesse.

Aus der DE 20 2004 012 014 U1 ist eine Biogasanlage mit einem Fermenter bekannt, die eine Fördervorrichtung zur Förderung der Biomasse von einem Biomassebunker zum Fermenter aufweist. Die Fördervorrichtung weist eine Förderschnecke auf, die das Biomassegut einem Zerkleinerer zuführen kann, von dem es aus einer weiteren Förderschnecke aufzugeben ist, die das zerkleinerte Gut dem Fermenter zuführt. Soll Biomassegut nicht zerkleinert werden, ist es mittels eines verstellbaren Leitblechs unter Umgehung des Zerkleinerers direkt der dem Fermenter zuführenden Förderschnecke aufzugeben. Nachteilig ist, daß das Biomassegut dem Zerkleinerer von oben mittels Schwerkraft aufzugeben ist, so daß es geschehen kann, daß das Biomassegut unkontrolliert durch beispielsweise witterungsabhängige Fremdeinflüsse ungewollt an dem Zerkleinerer vorbei fällt.

Es ist Aufgabe der vorliegenden Erfindung, eine Vorrichtung der eingangs genannten Art zu schaffen, bei der in einem verbesserten Maß Biomasseprodukte den Fermentereinheiten zur Verfügung zu stellen sind, die in einem verbesserten planbaren Maß einen nachfolgenden Gärungsprozeß gestalten.

Zur Lösung dieser Aufgabe zeichnet sich die Vorrichtung der eingangs genannten Art durch die im Patentanspruch 1 angegebenen Merkmale aus. Hinsichtlich wesentlicher vorteilhafter Ausgestaltungen dieser Vorrichtung wird auf die Ansprüche 2 bis 31 verwiesen.

Damit wird eine Vorrichtung zur Förderung von Biomassematerialien zur Verfügung gestellt, in deren Förderstufe, beispielsweise mit einer ersten Förderschnecke das Biomassematerial dem Zerkleinerer zuzuführen ist, von dem aus es der weiteren Förderstufe, beispielsweise einer zweiten Förderschnecke übergeben werden kann, und zwar so zerkleinert, wie dies gewünscht ist. Zusätzlich ist es mit dieser Vorrichtung möglich, die mithin eine Zentraleinheit im Rahmen der einem Fermenter vorgeschalteten Förderstrecke bilden kann, den Zerkleinerer zu umgehen, in dem das Material direkt dem weiteren Förderer, also z.B. der zweiten Förderschnecke, zugegeben wird. Dabei kann das Material, das jeweils aufgegeben wird, durchaus von einer einzigen vertikalen Förderstrecke der Vorrichtung zugeführt werden und z.B. über den ersten Förderer unter Umgehung des Zerkleinerers dem weiteren Förderer in der Vorrichtung übergeben werden. Diese ermöglicht, daß die Vorrichtung mit einer zentralen Zuführöffnung auskommt. Dadurch, daß der dem Zerkleinerer zugeordnete Förderer in einer Seitenwandung in dem Zerkleinerer mündet, fällt Fördergut nicht unkontrolliert infolge Schwerkraft in den Zerkleinerer, sondern wird in den Zerkleinerer zwangseingeführt. Dazu ist dem Förderer eine Preßschnecke vorgesehen, die in dem Seitenwandbereich des Zerkleinerers mündet, wodurch Anbackungen und Verstopfungen sicher vermieden wird. Zudem steht von Anfang an die volle Arbeitsbreite des Zerkleinerers zur Verfügung. Damit ist sicher planbar ein Förderprozeß zu vollziehen. Dies kann beispielsweise dadurch realisiert werden, in dem die beiden Förderer sich bereichsweise überlappen und untereinander in der Vorrichtung angeordnet sind und inzwischen den beiden Förderern ein Verschlußelement, beispielsweise eine verschiebbare Klappe, vorgesehen ist. Diese Klappe kann aufgesteuert werden, wonach das entsprechende aufgegebene Material z.B. an der Förderronde einer Förderschnecke vorbei in die darunter befindliche weitere Förderschnecke fällt und sodann unter Umgehung des Zerkleinerers in die weitere Förderschnecke hin zum Fermenter bewegt wird. Dabei kann die Klappe selbstverständlich auch auf ein vorbestimmbares Spaltmaß aufgesteuert werden, um eine gewisse Menge direkt in den weiteren Förderer gelangen zu lassen, aber auch einen gewissen Teil des aufgegebenen Biomassematerials dem Zerkleinerer zuzuführen. Ebenfalls ist es möglich, z.B. im Bereich einer zentralen Zuführöffnung weitere Anschlüsse vorzusehen, um beispielsweise durch Schwerkraft oder auch z.B. durch eine Zellenradschleuse z.B. Getreide, sei es trockenes Getreide, aber auch z.B. Mais mit einem gewissen Feuchtegrad, zuzugeben, um dieses über die Zerkleinerungswerkzeuge des Zerkleinerers zu führen, wobei in einem solchen Falle beispielsweise aufgrund der Zugabe von Mais es sich nicht um eine reine Trockenmahlung handelt, so daß auch Staubexplosionsrisiken vorgebeugt ist.

Ebenfalls ist es möglich, z.B. die Fördergeschwindigkeit der einzelnen Förderschnecken zu steuern, z.B. auch in Abhängigkeit des Füllungsgrades bzw. der abgenommenen Leistung in der Mühle, um einen dosierten Betrieb zu ermöglichen. Dies kann durch geeignete Meßwertgeber erfolgen, so daß von einer zentralen Steuereinheit aus entsprechende Einstellungen in der Vorrichtung insgesamt, also z.B. Geschwindigkeit der Förderschnecken, Auf- und Zu- bzw. Spaltsteuerung der Verschlußklappe, Geschwindigkeit der einzelnen Förderschnecken auch im Verhältnis zueinander, ein feindosierbarer regelbarer Betrieb ermöglicht ist.

Besonders vorteilhaft ist, wenn der Zerkleinerer eine Siebeinheit aufweist, die aus einer Außerbetriebsstellung in eine den Zerkleinerungswerkzeugen zugeordnete Betriebsstellung zurücküberführbar ist. Durch daß aus einer Außerbetriebsstellung in eine den Zerkleinerungswerkzeugen zugeordnete Betriebsstellung und zurücküberführbare Siebeinheit ist eine unmittelbar dem Zerkleinerer zugeordnete Einheit geschaffen, die in Abhängigkeit des aufzugebenden Biomassematerials eine Vorbehandlung ermöglicht, mit dem produzierbare Aufgabemasse für anschließende Fermentereinheiten zur Verfügung zu stellen sind. So ist es insbesondere ermöglicht, auf den Feuchtigkeitsgehalt des Biomassematerials Rücksicht zu nehmen, um beispielsweise die Siebeinheit in ihre Betriebsstellung zu überführen, in der sie den Zerkleinerungswerkzeugen insgesamt zugeordnet ist, wenn ein sehr trockenes Biomassematerial aufgegeben wird. Ist das Biomassematerial noch naß, kann die Siebeinheit in ihrer Außerbetriebsstellung überführt werden, so daß die Zerkleinerungswerkzeuge ohne zugeordnete Siebeinheit arbeiten können.

Bevorzugtermaßen ist die Siebeinheit auch als Verstellsieb ausgebildet und hat unterschiedliche Siebe mit entsprechenden Löchern, die relativ zueinander bewegt bewegbar sind, so daß die einander zugeordneten Sieblöcher aufgrund der Relativbewegung zueinander unterschiedlich große Öffnungssiebquerschnitte freigeben. Hinsichtlich derer Gestaltung wird auf die DE 102 45 447.7 A, die DE 102 45 445.0. A und die DE 102 45 446.9 A verwiesen, deren Inhalt ausdrücklich zum Offenbarungsgehalt dieser vorliegenden Patentanmeldung gemacht wird. Ebenfalls ist bevorzugt vorgesehen, daß der Zerkleinerer als Zerkleinerungsmühle mit zugeordneten Prallleisten ausgebildet ist, wobei die Pralleisten verstellbar sind, und zwar derart, daß die Pralleisten um eine exzentrisch angeordnete Schwenkachse schwenkbar ausgebildet sind. Damit ist auch steuerbar auf das Zerkleinerungs- bzw. Mahlgut Einfluß zu nehmen. Auch die Verstellung der Pralleisten kann von einer zentralen Steuereinheit erfolgen.

Der Förderer kann dabei mit Verstellsieben ausgerüstet sein, wie sie im einzelnen der DE-Patentanmeldung 103 35 881 A1 zu entnehmen ist.

Darüber ist es möglich, den Zerkleinerer (vorzugsweise die Mühle) mit einem Schneideinsatz zu versehen, der schwenkbar gelagerte Messer umfaßt, die jeweils mittels einer Druckfeder in Arbeitsposition gehalten werden. Bei Fremdkörpereinwirkung können die Messer einzelnd ausweichen und gehen danach sofort in ihre Arbeitsposition zurück. Ein solcher Schneideinsatz ist insbesondere bei Grassilage und langfasrigem Fördergut vorteilhaft. Bevorzugterweise ist der Schneideinsatz an einer Messertraverse gehaltert, die beweglich ist. Dazu können zwei Hydraulikzylinder vorgesehen sein, die die Messertraverse mitsamt dem Schneideinsatz in eine Außerbetriebsstellung und in eine Betriebsstellung überführen. Dazu ist ein Rotor des Zerkleinerers nicht abzuschalten.

Zudem ist es möglich, daß auf zwei Seiten des Zerkleinerers Einsätze vorgesehen sind, die Prallplatten tragen. Darüber hinaus ist es möglich, auf der einen Seite einen Einsatz mit Prallplatten und auf der anderen Seite einen Deckel mit einem Schneideinsatz vorzusehen, die jeweils an einer gelenkig gelagerten Gabeleinheit befestigt sind. Dieses ermöglicht einen schnellen Wechsel zwischen verschiedenen Werkzeugen, ohne daß Teile angehoben werden müssen.

Es ist auch möglich, daß Einsätze bzw. Deckel vorgesehen sind, die jeweils Schneidwerkzeuge haben, so daß eine Einheit mit Schneidwerkzeugen zum Einsatz kommen kann, während die andere während des Betriebes nachgeschliffen werden oder ein Messerwechsel vorgenommen werden kann. Die Einsätze bzw. Deckel mit Pralleisten sind symmetrisch gefertigt, so daß diese um 180° verschwenkt werden können, damit die Pralleisten beidseitig bis zur Verschleißgrenze nutzbar sind.

Darüber hinaus ist besonders bevorzugt vorgesehen, daß der Förderer eine als Trennschnecke ausgebildete Förderschnecke hat, indem diese einseitig gelagert ist und infolge ihres Eigengewichtes auf den Innenbereich des umgebenden Rohrwandungsbereiches des Förderers liegt. Dieser kann bereichsweise gelocht ausgebildet werden, so daß dieser Rohrwandungsbereich als Reibboden dient, wodurch Biomassematerial gepreßt wird. Oberhalb dieser seelenlosen Förder- bzw. Trennschnecke verbleibt ein Raum, in dem z.B. Verpackungsgut von Biomassematerialien, z.B. Verpackungsgut von in Supermärkten angebotene Gemüse, Salate, Obst, das zusammen mit der Verpackung in die Vorrichtung aufgegeben wurde und in der Zerkleinerungseinheit soweit aufgeschlagen wurde, daß das abgepackte Gut (mit überschrittenem Haltbarkeitsdatum) herausgebracht wurde, gleichwohl die Verpackung zwar aufgeschlagen aber noch nicht zerkleinert weitergefördert wird, so daß die Biomasse getrennt von der Verpackung weiterverarbeitet wird und das Verpackungsgut in dem oberen Förderkanal bis zu einer endseitigen Förderöffnung im Förderer transportiert werden und dort separiert einer Abgabestation zuzuführen ist. Damit ist mit der Vorrichtung nach der Erfindung vollautomatisch ein Trennprozeß durchzuführen, wobei aufgrund der Beaufschlagung mittels des Zerkleinerers von beispielsweise Obst (mit harter Schale) wie Orangen, Grapefruits derart aufbereitet werden, so daß es nach der anschließenden Zuführung über den Reibboden des Förderers im Fermenter einem kurzfristigem Gärprozeß unterliegt. Damit ist der Wirkungsgrad z.B. einer Biogasanlage mit Vergärung von Biomassematerialien wesentlich zu erhöhen. Aufgrund der in der Vorrichtung vorgenommenen Trennung sind die Gesamtverwertungskosten, der Logistikaufwand zur Verwertung von z.B. verpacktem Obst und Gemüse mit überschrittenem Haltbarkeitsdatum wesentlich verbessert.

Insgesamt ist damit eine Vorrichtung zur Verfügung gestellt, mit der in einer zentralen Fördereinheit die unterschiedlichsten Betriebsparameter der aufzugebenen Biomassematerialien exakt einzustellen und zu regeln sind. So lassen sich ansonsten auch nur schwer in einem Gärprozeß verwertbare Biomassematerialien einsetzen, indem sie eine entsprechende Vorbehandlung erfahren bzw. mit entsprechend anderen passenden Biomasseprodukten in der Vorrichtung vermischt werden. Damit ist es ermöglicht, mit einer gut händelbaren und im übrigen in einer auch baulich überschaubaren Zentraleinheit Biomassematerialien dem Gärprozeß zuzuführen, die mit bekannten Anlagen nicht oder nur teilweise verwertet werden können. Darüber hinaus ist in optimierter Weise auf den Gärprozeß Einfluß zu nehmen, so daß in reproduzierbarer und optimierter Weise z.B. eine Biomasseanlage eingestellt und betrieben werden kann.

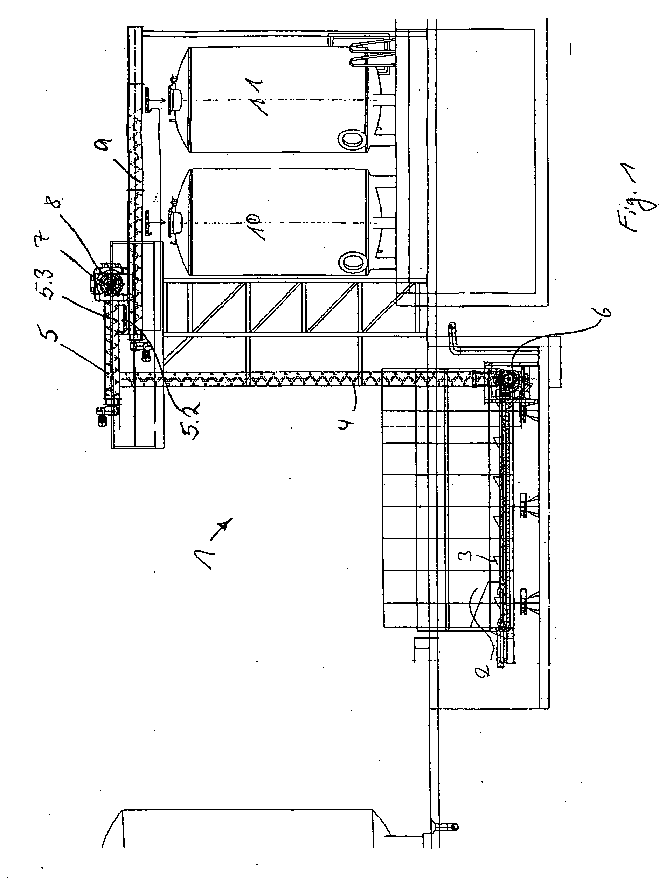

- Fig. 1: eine schematische Seitengesamtansicht einer Vorrichtung zur Förderung von Biomaterialien in Fermentereinheiten;
- Fig. 2: ausschnittsweise das Gehäuse einer Zerkleinerungsvorrich- tung mit zugeordneter Siebeinheit in der Außerbetriebsstellung der Siebeinheit in einer perspektivischen Darstellung;
- Fig. 3: eine zu Fig. 2 analoge Darstellung ohne die Darstellung der Siebflächen;
- Fig. 4: eine zu den Fig. 2 und 3 analoge Darstellung mit Darstellung der Zerkleinerungswerkzeuge und des Antriebes der Zerkleine- rungswerkzeuge;
- Fig. 5: das Ausführungsbeispiel nach Fig. 4 in einer anderen perspek- tivischen Darstellung mit Darstellung der Abführöffnung der Zerkleinerungsvorrichtung;
- Fig. 6: vergrößert die Zentralvorrichtung 1.1 in Fig. 1;
- Fig. 7: vergrößert ausschnittsweise einen Bereich des Zerkleinerers mit seinen verstellbaren Pralleisten;

- Fig. 8: eine Seitenansicht (geschnitten) auf ein weiteres Ausführungs- beispiel eines Zerkleinerers mit vorgesehenem Schneideinsatz;
- Fig. 9: den Schneideinsatz nach Fig. 8 vergrößert;
- Fig. 10: eine Draufsicht auf den Zerkleinerer nach Fig. 8;
- Fig. 11 1: eine zu Fig. 8 analoge Darstellung mit einer alternativen Anord- nung eines Messereinsatzes;
- Fig. 12: eine Draufsicht auf das Ausführungsbeispiel nach Fig. 11;
- Fig. 13: eine zu Fig. 8 und Fig. 11 analoge Darstellung eines weiteren alternativen Ausführungsbeispiels des Zerkleinerers;
- Fig. 14: eine Draufsicht auf das Ausführungsbeispiel nach Fig. 13;
- Fig. 15: eine perspektivische Darstellung des Zerkleinerers mit beidsei- tig am Gehäuse angelenkten Pralleisteneinsätzen;

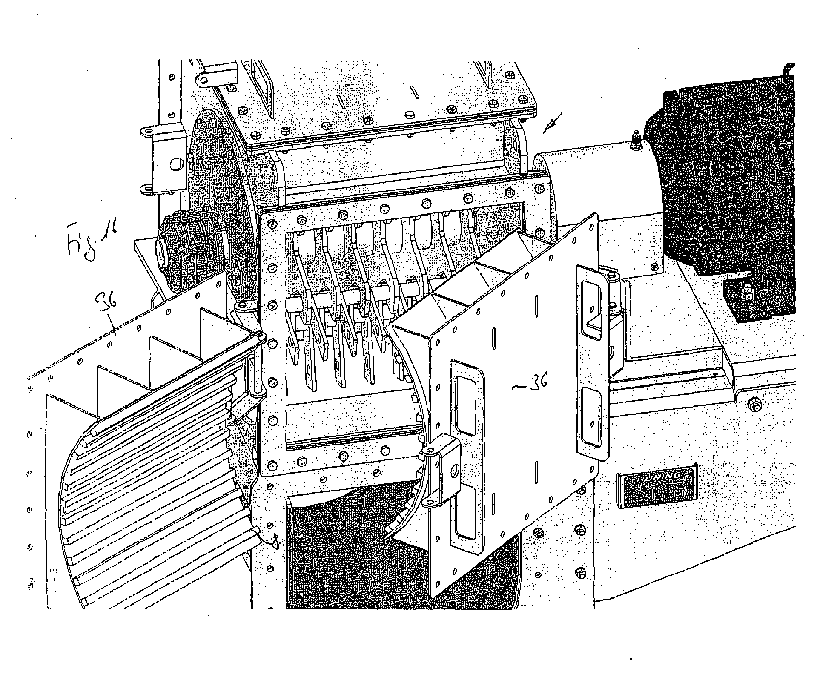

- Fig. 16: das Ausführungsbeispiel nach Fig. 15 mit einem halb einge- klappten Pralleisteneinsatz und einem geöffneten Pralleisten- einsatz;
- Fig. 17: eine Seitenansicht des dem Zerkleinerer nachgeordneten För- derers;
- Fig. 18: eine Ansicht von unten auf das Ausführungsbeispiel nach Fig. 16 und Fig. 17;
- Fig. 19: eine Ansicht auf die Stirnfläche des Förderers im Bereich sei- ner Ausgabeöffnung; und
- Fig. 20: eine Ansicht einer Biomasseverwertungsanlage z.B. zur Er- zeugung von Biogas mit einer Vorrichtung nach der Erfindung.
In der Zeichnung sind grundsätzlich gleichwirkende Teile mit übereinstimmenden Bezugsziffern versehen.

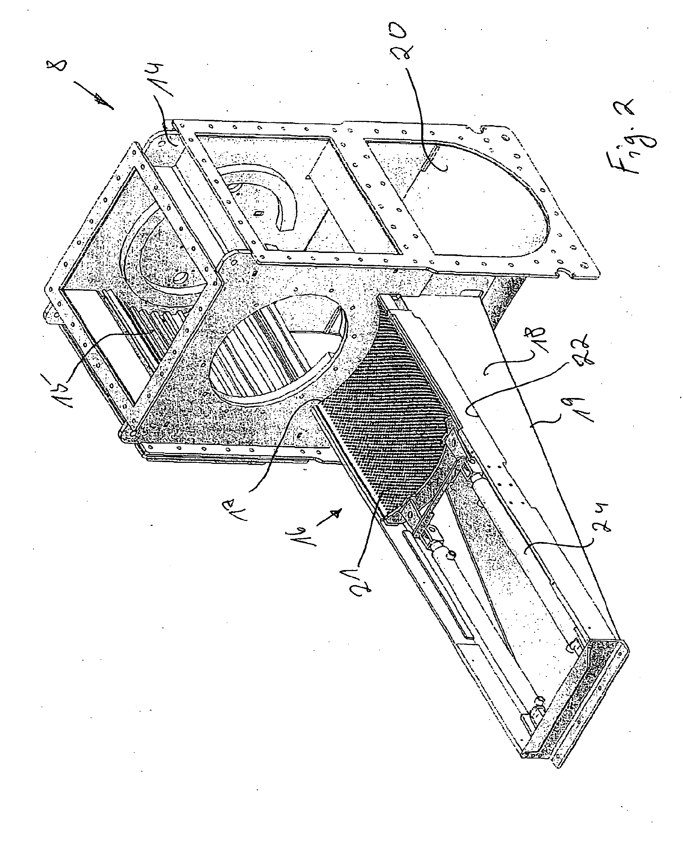

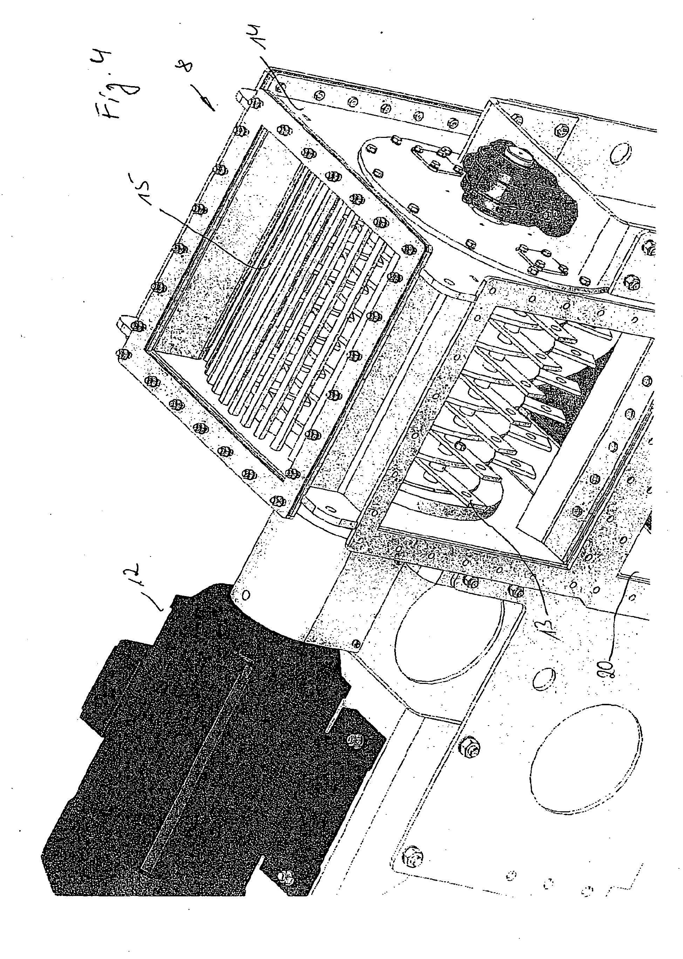

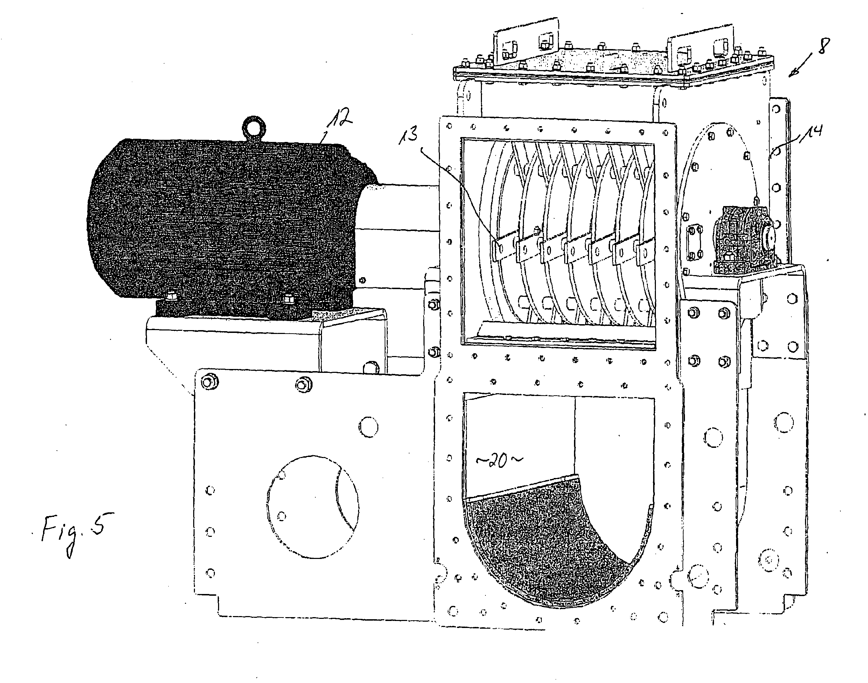

In der Zeichnung ist allgemein mit 1 eine Anlage zur Förderung von Biomaterialien beziffert. Diese umfaßt eine Aufgabeeinheit 2, auf die Biomasseausgangsmaterialien aufzugeben sind. Über eine bewegbare Förderrampe 3 wird diese einer Vertikalförderschnecke 4 als Vertikalförderstufe zugeführt. Diese mündet in eine Vorrichtung 1.1 nach der Erfindung mit einer Förderschnecke 5, die horizontal ausgerichtet ist. Sowohl bei 6 als auch bei 7 ist ein Zerkleinerer vorgesehen sein. Im nachfolgenden wird bei 7 der Zerkleinerer 8 der Vorrichtung 1.1 in Gestalt einer Mühle näher erläutert. Über eine zweite Förderschnecke 9 der Vorrichtung 1.1 und auch Förderschnecken weiterer Förderstufen können die zerkleinerten, nicht zerkleinerten und/oder vermischten Biomassematerialien jeweils einer Fermentereinheit 10 und 11 zugeführt werden, z.B. für einen nachfolgenden Gärprozeß. Von ihren Bauteilen ist die Zerkleinerungseinheit 8 bei 7 in den Fig. 2 bis 5 näher erläutert. Der Zerkleinerer 8 hat (Fig. 4) über einen Motor 12 in Rotationsbewegung versetzbare Zerkleinerungswerkzeuge 3 in Gestalt von Schlagleisten. Oberhalb des Gehäuses 14, und zwar über einen Deckel verschließbar, sind Pralleisten 15 den Zerkleinerungswerkzeugen zugeordnet.

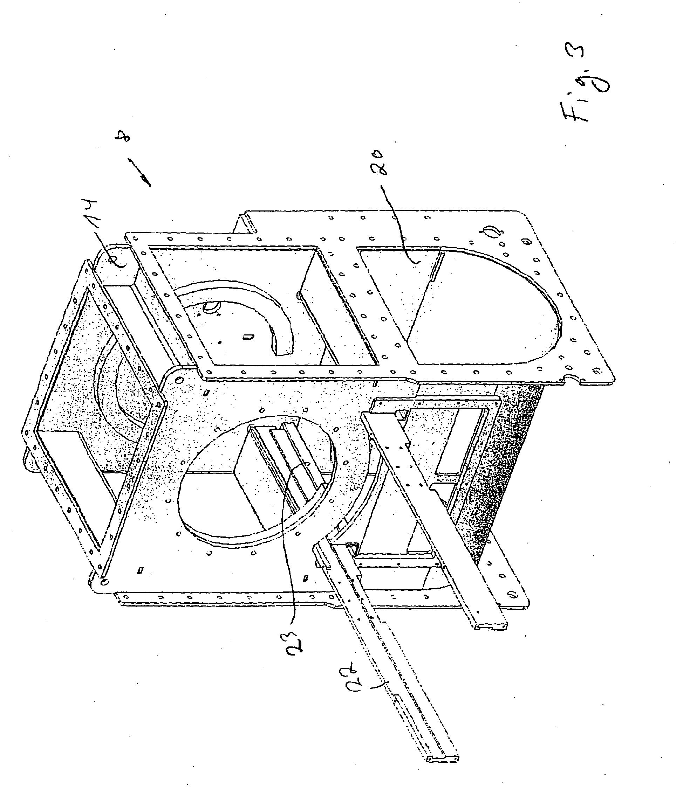

In Fig. 2 näher dargestellt (ohne Zerkleinerungswerkzeuge und ohne Deckel), ist den Zerkleinerungswerkzeugen eine Siebeinheit 16 zugeordnet. Diese Siebeinheit ist in das Gehäuse 14 zu bewegen und in der Fig. 2 in einer Betriebsstellung gezeigt, in der sie sich außerhalb dieses Gehäuses 14 befindet. Dazu ist in dem Gehäuse eine Öffnung 17 vorgesehen. Unterhalb der Öffnung 17 ist eine wannenartige Einheit 18 mit einer unteren Schüttfläche 19 angebaut, wobei die Schüttfläche 19 unmittelbar in die schachtartige Austragöffnung 20 des Gehäuses 14 des Zerkleinerers 8 mündet. Die Siebeinheit 16 hat ein Sieb 21, das leicht gekrümmt ist. Dieses Sieb ist, wie näher aus Fig. 3 hervorgeht, an einem Schlitten 22 geführt, der entlang von Führungen 23 über Hydraulikzylinder 24 zu bewegen ist, und zwar aus der aus Fig. 2 ersichtlichen Außerbetriebsstellung hinein in eine Stellung, in der die Siebeinheit nach unten in die Zerkleinerungswerkzeuge vollständig umgibt, so daß der darunter liegende Schacht mit der Schachtöffnung 20 für das Biomasseprodukt erst erreichbar ist, wenn das Sieb passiert wurde. Dies kann so gestaltet sein, wie in den eingangs genannten Patentanmeldungen näher beschrieben wurde. Danach kann es eine Verstellsiebeinheit sein mit zwei oder mehr aufeinander liegenden Siebelementen, mit jeweils einer Mehrzahl von Sieblöchern, die in relative Lagen zueinander zu bringen sind, so daß dadurch das Lochmuster, die Lochgröße und/oder die Lochstruktur zu verändern ist.

Im einzelnen nicht näher dargestellt ist ein Feuchtesensor, der die Feuchte des aufzugebenden Biomassematerials ermittelt und an eine elektrische Stelleinheit weitergibt, die in Abhängigkeit des Feuchtegehaltes des Biomassematerials die Stellpositionen der Siebeinheit durchführt, gleichzeitig jedoch auch für eine Verstellung der Verstellsiebelemente zueinander Sorge tragen kann. Zusätzlich können noch weitere Bedienelemente vorhanden sein, um beispielsweise vorprogrammierbar oder manuell die Überführung der Siebeinheit in ihre Betriebsstellung und zurück zu bewerkstelligen oder aber auch beispielsweise Lochgröße, Lochstruktur und dergleichen zu verändern.

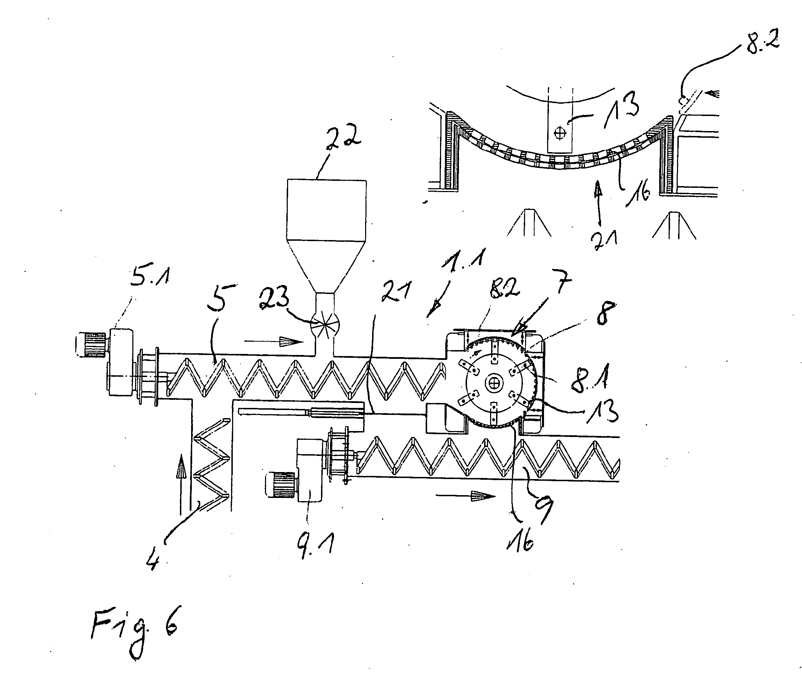

In Fig. 6 ist die Vorrichtung 1.1 mit ihrer Mühle 8 als Zerkleinerer und den beiden einander zugeordneten horizontal ausgerichteten Förderschnecken 5 und 9 als Förderer dieser Förderstufe näher veranschaulicht. Über die Vertikalförderschnecke 4 kann Biomassematerial an die erste Förderschnecke 5 gegeben werden, die von einem Motor 5.1 angetrieben ist. Vor dem Zerkleinerer 8 mit seinen Schlagleisten 8.1 und seinen Pralleisten 8.2. ist ein Verschließelement 21 vorgesehen, das hydraulisch betätigt auf und zu gefahren werden kann, aber auch auf einen Spaltmaß mit einem gewissen Öffnungsquerschnitt aufzufahren ist. Ist das Verschließelement 21 in Gestalt der Verschlußklappe geschlossen, wie in Fig. 6 dargstellt, wird das gesamte Material, das von der Förderschnecke 5 gefördert wird, dem Zerkleinerer 8 zugeführt. Ist die Verschlußklappe 21 geöffnet, wird das meiste von der Förderschnecke 5 geförderte Material durch die dann nicht mehr verschlossene Öffnung an die unter der Förderschnecke 5 gelegene Förderschnecke 9 weitergegeben, die von ihrem Motor 9.1 angetrieben ist. Damit ist ganz oder teilweise der Zerkleinerer 8 zu umgehen.

Vor der Verschlußklappe 21 ist noch ein Vorratsbehälter 22 vorgesehen, über den mittels eines Dosierers 23 dosiert beispielsweise Zuschlagsstoffe beigegeben werden können. Darüber kann beispielsweise Getreide aber auch sonstiges Material aufgegeben werden. Sämtliche Teile wie Schnecken 5 und 9, Zerkleinerer, Dosierer 23 sind über eine Zentralsteuereinheit (nicht näher gezeigt) gesteuert, so daß diese Vorrichtung so automatisiert zu fahren ist, daß in Abhängigkeit der aufgegebenen Biomassematerialien, aber auch in Abhängigkeit gefahrener Betriebszustände, die über geeignete Meßwertgeber zu erfassen sind, ein automatischer, aber voreinstellbarer Betrieb ermöglicht ist.

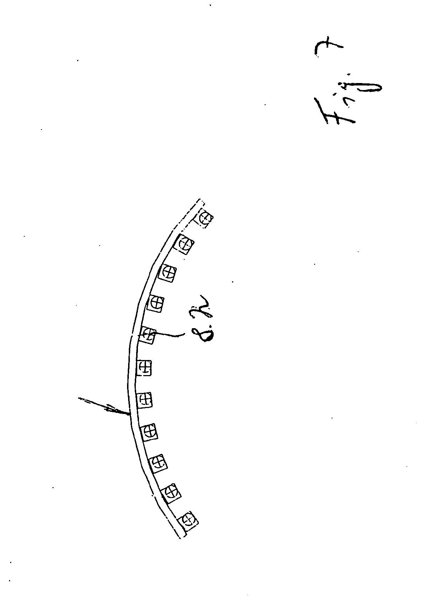

Die Pralleisten 8.2 sind, wie Fig. 7 zu entnehmen sind, schwenkbar ausgebildet. Dazu haben sie eine exzentrisch angeordnete Schwenkachse 8.3, um die sie verschwenkt werden können, woraufhin die Pralleisten 8.2 einen unterschiedlichen Abstand zu den Schlagwerkzeugen 8.1 einnehmen.

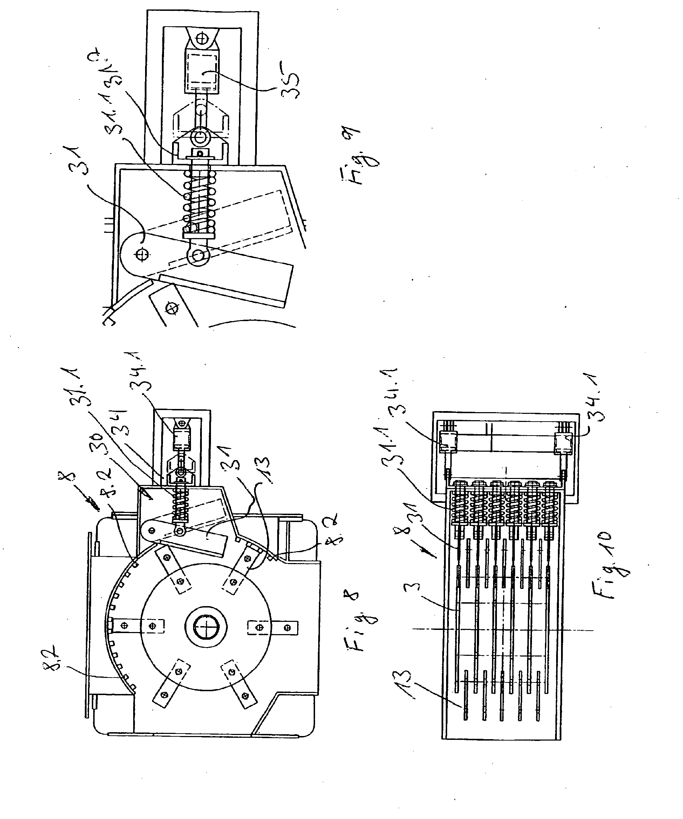

In dem in den Figuren 8 bis 14 gezeigten Ausführungsbeispielen sind zusätzlich zu den Pralleisten 2 und den Zerkleinerungswerkzeugen 13 noch jeweils ein Schneidmessereinsatz vorgesehen. In dem Ausführungsbeispiel nach den Figuren 8 bis 10, ist im rechten Bereich des Zerkleinerers eine Messertraverse 34 vorgesehen, an der der Schneidansatz 30 mit den Schneidmessern 31 abgestützt ist. Jedem Schneidmesser 31 ist eine Druckfeder zugeordnet, die sich einerseits am Schneidmesser 31 und anderenends an der Traverse 34 abstützt. Über die Druckfeder wird das Schneidmesser 31 in der durch die durchgezogenen Linien angezeigten Betriebsstellung gehalten. Bei Fremdkörpereinwirkung kann jedes einzelne Schneidmesser 31 gegen die Kraft der Druckfeder ausweichen. Über einen Hydraulikzylinder 34.1 kann das Schneidmesser in eine Außerbetriebsstellung gebracht werden, wie strichpunktiert in den Fig. 8 und Fig. 9 angedeutet.

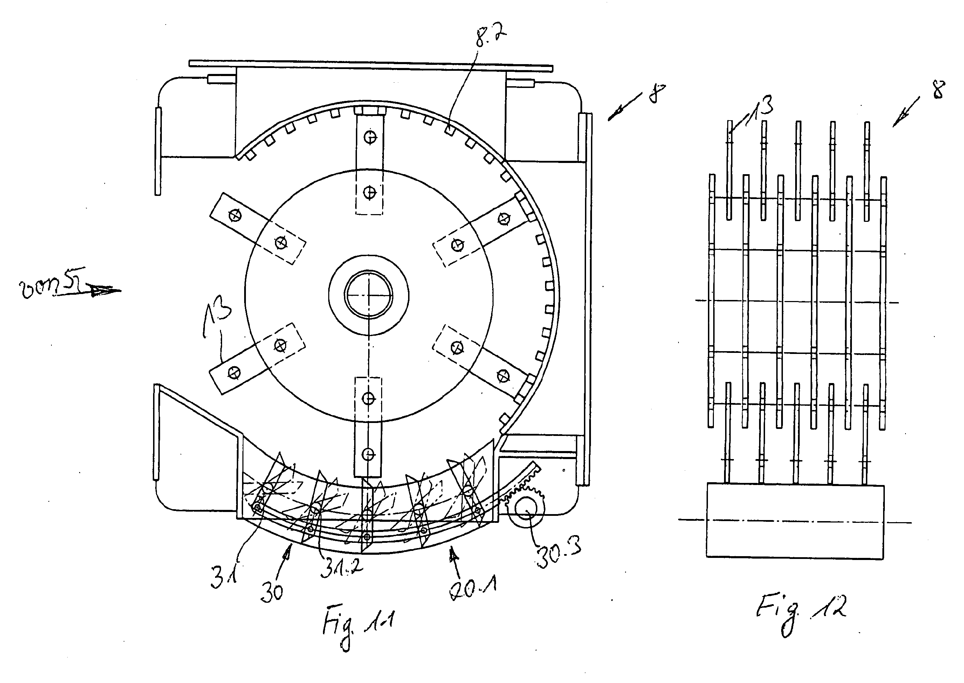

In dem alternativen Ausführungsbeispiel nach den Figuren 11 und 12 sind im Bereich der Öffnung zum Schacht 20.1 (Fig. 2) des Zerkleinerers 8 Schneidmesser 31 des Schneidmessereinsatzes vorgesehen, die um Achsen schwenkbar sind. Die Messer beherrschen die Auslauföffnung zum Schacht 20.1 und können verstellt werden, so daß diese Auslauföffnung verstellbar.ist. Mittels eines Stellmotorantriebes 30.3 kann mittels der Zahnstange die Feststellung durchgeführt werden. In diesem Ausführungsbeispiel ist auch zu sehen, daß durch den seitlichen Eintrag (dargestellt mit dem Pfeil "von 5 kommend") das zugeführte Material den Zerkleinerungsprozeß der Mühle zwangseingeführt wird durch die in dem Förderer 5 vorgesehenen Förderschnecke. Dies ist wesentlich günstiger, als das Material beispielsweise infolge von Schwerkraft in eine Mühle hineinfallen zu lassen.

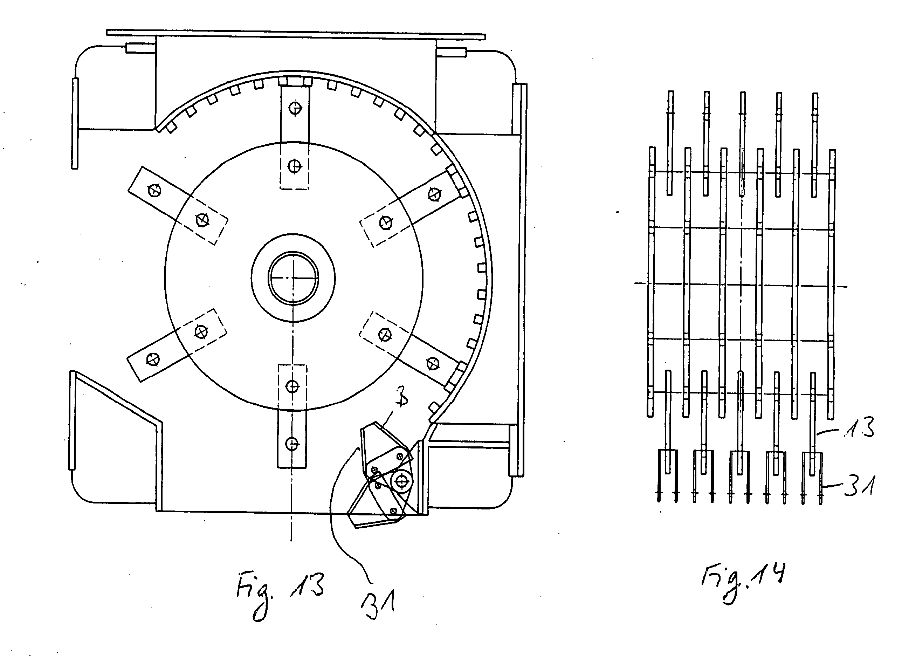

In dem Ausführungsbeispiel nach den Fig. 13 und Fig. 14 ist das Messer 31 in der oberen Darstellung gezeigt, veranschaulicht durch den Buchstaben B in der Betriebsstellung. Dieses kann abgeklappt werden, wenn es für den Betrieb nicht benötigt wird und wird beispielsweise automatisch bei der Zuführung von z.B. Grassilage in die Betriebsstellung verschwenkt. Dazu sind geeignete Schwenkantriebe vorzusehen.

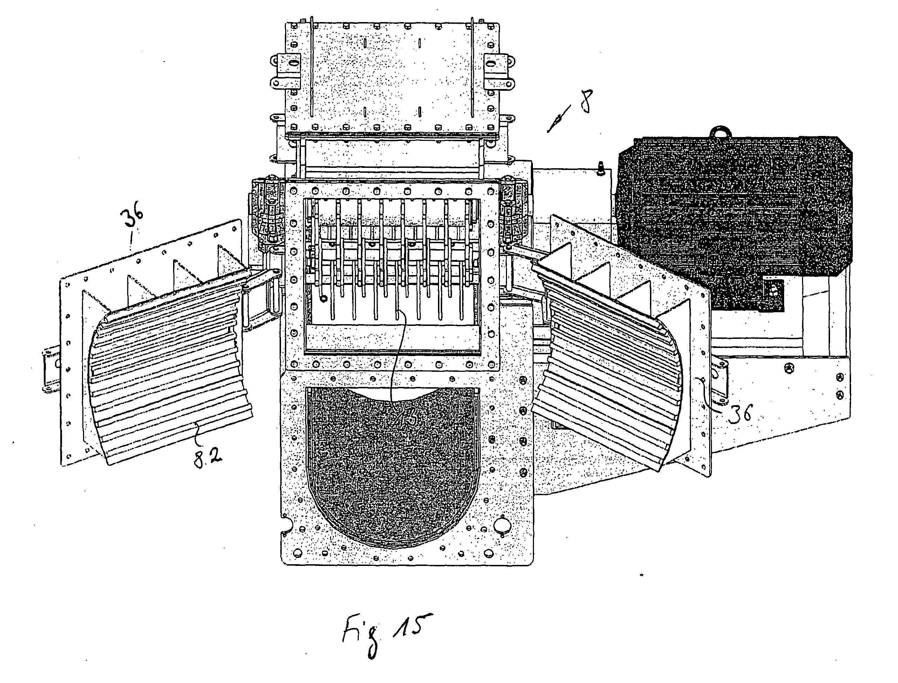

Fig. 15 zeigt eine Ausführung, bei der beidseits des Maschinengehäuses Praliplatteneinsätze 36 mit Pralleisten 8.2 vorgesehen ist. Es ist auch möglich, auf der einen Seite beispielsweise einen Einsatz mit Prallelementen 8.2 und auf der anderen Seite einen Deckel mit Schneidelementen vorzusehen. Diese sind an einer gelenkig gelagerten Gabeleinheit befestigt. Dabei ist es möglich, z.B. einen Einsatz 36 einzuschwenken, der für den laufenden Betrieb benötigt wird. Ein anderer kann offen bleiben, um ihn beispielsweise mit verschiedenen Werkzeugen zu bestücken. Beim Einsatz von Messern können diese z.B. nachgeschliffen werden. Die Deckel mit den Pralleisten 8.2 sind symmetrisch gefertigt, so daß diese um 180° gedreht werden können, damit die Pralleisten beidseitig bis zur Verschleißgrenze benutzt werden können.

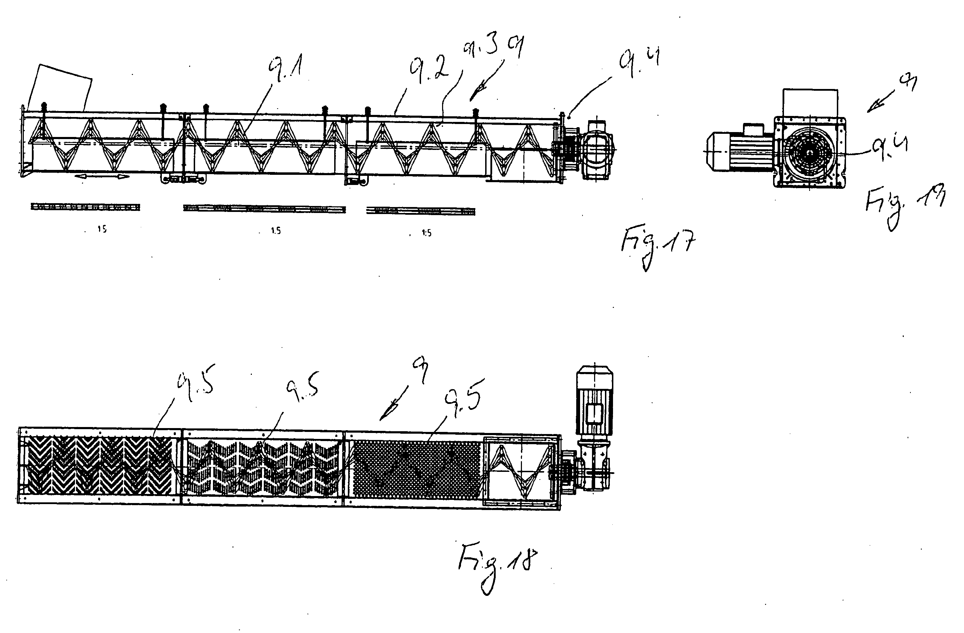

Die Fig. 17, Fig. 18 und Fig. 19 veranschaulichen näher die nähere Ausgestaltung des Förderers 9. Dieser schließt sich unmittelbar an den Zerkleinerer 8 an (siehe unter anderem Fig. 6) und hat im Inneren eine einseitig gelagerte Förderschnecke 9.1 (seelenlose Schnecke), die als Trennschnecke ausgebildet ist. Diese läuft in einem innerhalb des Förderrohres 9.2 ausgebildeten Raum und hat an ihrer Unterseite insgesamt drei Verstellsiebe. Diese können so gestaltet sein, wie dies näher in der DE 103 35 881 A1 beschrieben ist. Alternativ ist es auch möglich, die Wandung des Förderrohres mit einem Lochmuster zu versehen. Aufgrund der einseitigen Lagerung bilden die einzelnen Verstellsiebe bzw. die Innenmantelfläche des Förderrohres mit dem Lochmuster einen Reibboden, da die einseitig gelagerte Schnecke infolge ihres Eigengewichtes dort aufliegt. Im oberen Bereich, nicht im einzelnen näher veranschaulicht, jedoch durch die Bezugsziffer 9.3 angedeutet, ist ein Raum, in dem beispielsweise aufgeschlagenes Verpackungsmaterial von z.B. Verpackungseinheiten "Obst, Gemüse, Salat" aus Supermärkten mit überschrittenem Haltbarkeitsdatum gesondert transportiert werden kann bis hin zu einer Austragöffnung 9.4, so daß einerseits durch die Verstellsiebe 9.5 das Biomassematerial von der Förderschnecke hindurchgepreßt wird, oben jedoch das lose geführte Verpackungsmaterial getrennt herausgebracht wird. Die Verstellsiebe haben dabei ein Siebmaß, das von Feinmittel bis grob reicht.

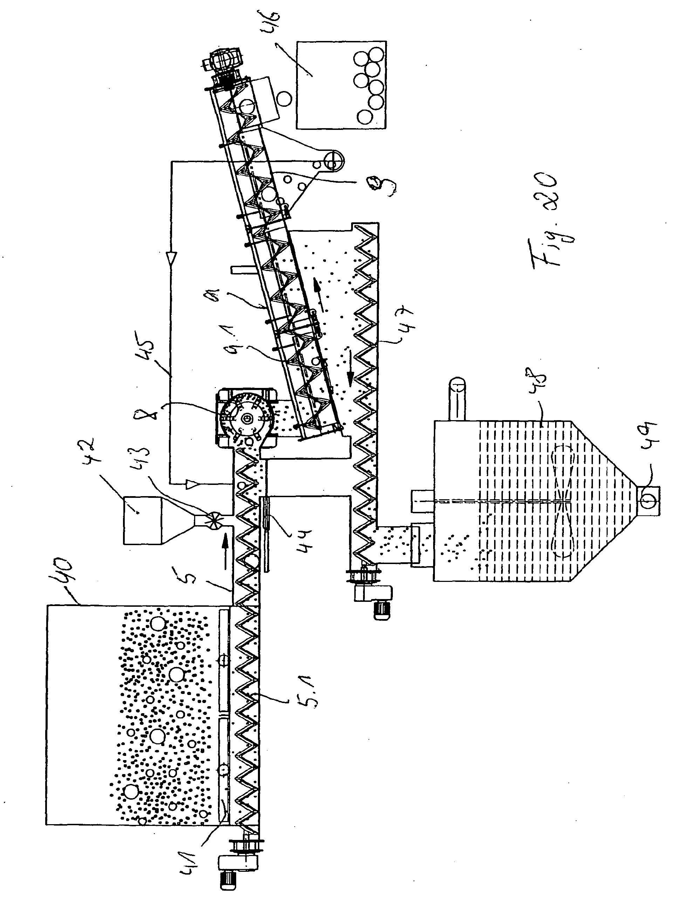

In Fig. 20 ist ganz allgemein noch einmal noch einmal eine Vorrichtung bzw. Verwertung von Biomasseverwertungsmaterialien mit einer Vorrichtung nach der Erfindung unter Einschluß des Zerkleinerers dargestellt. Diese ist in dem dargestellten Ausführungsbeispiel einer Hammermühle 8, zumindest ist ein Annahmebehälter 40 vereinschaulicht, der einen Schubboden 41 aufweist, dargestellt in dem Annahmebehälter ist Biomasse mit Fremdstoffen. Dies können beispielsweise verpacktes Gemüse, verpacktes Obst, verpackter Salat und dergleichen sein, der von Supermärkten angeliefert wird, da das entsprechende Haltbarkeitsdatum überschritten ist. Dieses Biomassematerial wird verpackt der Anlage zugeführt, wobei vor der Zuführung in einem nicht dargestellten Fermenter selbstverständlich das Verpackungsmaterial von der Biomasse zu trennen ist. Das Biomassematerial gelangt in den Förderer 5 mit der inneren Förderschnecke 5.1. Zu diesem Zeitpunkt ist das Biomassematerial noch mit dem Verpackungsgut umgeben. Es ist ein Kleinkomponentenzugabebehälter 42 vorgesehen, über den und eine Dosiereinrichtung 43 Zuschlagstoffe beigegeben sind. Es ist ein Beipaßschieber 44 dargestellt, der im Falle des verpackten Gemüses und dergleichen jedoch geschlossen wird, so daß über eine Zwangseinführung seitlich in die Hammermühle 8 mittels der als Spiralschnecke ausgebildeten Förderschnecke 5.1 das Material dem Zerkleinerungsprozeß zugeführt wird. Durch die Hammermühle wird das Verpackungsmaterial aufgeschlagen, so daß das eingeführte Material herausfallen kann. Durch das Aufschlagen des Verpackungsmaterials innerhalb der Hammermühle wird das Verpackungsmaterial jedoch nicht derart zerkleinert, daß es fein verteilt vorliegt. Vielmehr bleibt es als grobganzes erhalten und kann nun dem Förderer 9 zugegeben werden, indem die seelenlose Förderschnecke 9.1 vorgesehen ist. An Preßschnecke 5.1 ist noch ein Rücklauf für aussortierte Grobstoffe im Biomassematerial vorgesehen, um dieses einem erneuten Prozeß zuzuführen. Über die Förderschnecke 9.1 gelangen die Grobstoffe wieder zum Verpackungsmaterial in einem Abfallbehälter 46. Das Biomassematerial, was durch die Lochwandung bzw. Verstellsiebe des Förderers 9 hindurchkommt, gelangt zu einem weiteren Förderer 47, der die Biomasse einem Anmischbehälter 48 zuführt, von dem es über eine Förderleitung 49 dem nicht dargestellten Fermenter zugeführt wird.

## Patentansprüche

1. Vorrichtung zur Förderung von Biomassematerialien in z.B. einen Fermenter (10,11) mit zumindest einer beispielsweise eine Förderschnecke als Förderer (5) aufweisenden Förderstufe mit einem der Förderstufe zugeordneten, Zerkleinerungswerkzeuge aufweisenden Zerkleinerer (8), beispielsweise einer Mühle, wobei die Förderstufe einen weiteren Förderer (9) aufweist, durch den Biomassematerial unter Umgehung des Zerkleinerers förderbar ist und dem das von dem Zerkleinerer (8) kommendes Biomassematerial zuführbar ist, **dadurch gekennzeichnet, daß** der dem Zerkleinerer zugeordnete Förderer (5) in einem Bereich einer Seitenwandung in den Zerkleinerer (8) mündet und über den Förderer (5) eine Zwangseinführung über eine im Förderer (5) vorgesehene Preßschnecke in den Zerkleinerer (8) erfolgt.

2. Vorrichtung nach Anspruch 1, **dadurch gekennzeichnet, daß** die beiden Förderer (5, 9) der Förderstufe zumindest bereichsweise parallel zueinander ausgerichtet sind.

3. Vorrichtung nach Anspruch 1 oder 2, **dadurch gekennzeichnet, daß** die beiden Förderer (5, 9) der Förderstufe in Förderrichtung des Biomassematerials in Reihe geschaltet sind.

4. Vorrichtung nach einem der Ansprüche 1 bis 3, **dadurch gekennzeichnet, daß** zwischen den Förderern (5, 9) der Förderstufe eine Bypaßöffnung (5.3) vorgesehen ist, die über ein Verschlußelement (5.2) freizugeben und zu verschließen ist.

5. Vorrichtung nach einem der Ansprüche 1 bis 4, **dadurch gekennzeichnet, daß** einem der beiden Förderer (5, 9) über eine Zuführöffnung Beimengungsmaterial zuführbar ist.

6. Vorrichtung nach einem der Ansprüche 1 bis 5, **dadurch gekennzeichnet, daß** die Fördergeschwindigkeit der Förderer (5. 9) in Abhängigkeit von einstellbaren Betriebsparametern regelbar ist.

7. Vorrichtung nach einem der Ansprüche 1 bis 6, **dadurch gekennzeichnet, daß** der weitere Förderer (9) der Förderstufe in einem Schacht (20.1) unterhalb des Zerkleinerers (1) und unterhalb eines Bereiches des ersten Förderers (5) der Förderstufe in der Vorrichtung vorgesehen ist.

8. Vorrichtung nach einem der Ansprüche 1 bis 7, **dadurch gekennzeichnet, daß** der Zerkleinerer eine Siebeinheit (21) aufweist, die aus einer Außerbetriebsstellung in eine den Zerkleinerungswerkzeugen (13) zugeordnete Betriebsstellung und zurück überführbar ist.

9. Vorrichtung nach einem der Ansprüche 1 bis 8, **dadurch gekennzeichnet, daß** die Siebeinheit (16) ein translatorisch bewegbares Sieb (21) aufweist.

10. Vorrichtung nach Anspruch 9, **dadurch gekennzeichnet, daß** das Sieb (21) eine gekrümmte Siebfläche aufweist.

11. Vorrichtung nach einem der Ansprüche 8 bis 10, **dadurch gekennzeichnet, daß** in ihrer Betriebsstellung die Siebeinheit (16) die Zerkleinerungswerkzeuge (13) bereichsweise begrenzt und den Zerkleinerer (8) bereichsweise nach unten zu einem Schacht (20.1) hin umgibt.

12. Vorrichtung nach einem der Ansprüche 1 bis 11, **dadurch gekennzeichnet, daß** die Siebeinheit (16) einen Siebschlitten (22) aufweist.

13. Vorrichtung nach einem der Ansprüche 1 bis 12, **dadurch gekennzeichnet, daß** der Siebschlitten (22) entlang von Führungen (23) bewegbar ist.

14. Vorrichtung nach Anspruch 12 oder 13, **dadurch gekennzeichnet, daß** das Sieb (21) von Hydraulik- oder Pneumatikzylindern (24) betätigbar ist.

15. Vorrichtung nach einem der Ansprüche 1 bis 14, **dadurch gekennzeichnet, daß** die Siebeinheit (16) ein in eine im Gehäuse des Zerkleinerers vorgesehene Öffnung einfahrbares Sieb (21) aufweist und durch diese Öffnung in ihre Betriebsstellung und ihre Außerbestriebsstellung außerhalb des Gehäuses überführbar ist.

16. Vorrichtung nach einem der Ansprüche 1 bis 15, **dadurch gekennzeichnet, daß** die Siebeinheit (16) zwei aufeinander liegende Siebelemente aufweist, die jeweils eine Mehrzahl von Sieblöchern aufweisen, die abhängig von der relativen Lage der Siebelemente zueinander Sieböffnungen ausbilden und die relative Lage der Siebelemente zueinander veränderbar ist.

17. Vorrichtung nach einem der Ansprüche 1 bis 16, **dadurch gekennzeichnet, daß** der Siebeinheit (16) ein Feuchtesensor zugeordnet ist und eine motorische Stelleinheit für die Überführung der Siebeinheit (16) aus ihrer Außerbetriebsstellung in ihre Betriebsstellung und zurück in Abhängigkeit des Ergebnisses der vom Feuchtesensor ermittelten Feuchte des Biomassematerials betätigbar ist.

18. Vorrichtung nach einem der Ansprüche 1 bis 17, **dadurch gekennzeichnet, daß** der Zerkleinerer (8) als Zerkleinerungsmühle mit Schlagleisten (8.1) und zugeordneten Pralleisten (8.2) ausgebildet ist.

19. Vorrichtung nach Anspruch 18, **dadurch gekennzeichnet, daß** die Pralleisten (8.2) verstellbar ausgebildet sind.

20. Vorrichtung nach dem Anspruch 19, **dadurch gekennzeichnet, daß** die Pralleisten (8.2) eine exzentrisch angeordneten Schwenkachse haben und verschwenkbar ausgebildet sind.

21. Vorrichtung nach einem der Ansprüche 1 bis 20, **dadurch gekennzeichnet, daß** dem Zerkleinerer (8) einen den Zerkleinerungswerkzeugen (13) zugeordneten Schneideinsatz (30) aufweist.

22. Vorrichtung nach Anspruch 21, **dadurch gekennzeichnet, daß** der Schneideinsatz (30) schwenkbar gelagerte Schneidmesser (31) aufweist.

23. Vorrichtung nach Anspruch 22, **dadurch gekennzeichnet, daß** die Schneidmesser (31) jeweils mittels einer Druckfeder (31.1) in Arbeitsposition haltbar und bei Fremdkörperbeaufschlagung einzeln ausweichbar gelagert sind.

24. Vorrichtung nach einem der Ansprüche 21 bis 23, **dadurch gekennzeichnet, daß** der Schneideinsatz (30) an einer beweglichen Messertraverse (31.2) abstützbar und über die Messertraverse (31.2) und eine Betätigungsvorrichtung (35) in eine Betriebsstellung und in eine Außerbetriebsstellung überführbar ist.

25. Vorrichtung nach einem der Ansprüche 1 bis 24, **dadurch gekennzeichnet, daß** die Pralleisten (8.2) an einem Pralleisteneinsatz (36) vorgesehen, der schwenkbar einem Gehäuse des Zerkleinerers (8) angelenkt ist und in eine Betriebsstellung und in eine Außerbetriebsstellung überführbar ist.

26. Vorrichtung nach Anspruch 25, **dadurch gekennzeichnet, daß** zumindest zwei Pralleisteneinsätze (36) vorgesehen sind, von denen teilweise der eine oder der anderere in die Betriebsstellung überführbar ist.

27. Vorrichtung nach einem der Ansprüche 1 bis 26, **dadurch gekennzeichnet, daß** die Vorrichtung auf der einen Seite einen Pralleisteneinsatz (36) und auf der anderen Seite ein Schneidwerkzeugeinsatz aufzeigt, die wahlweise in ihre Betriebsstellung überführbar sind.

28. Vorrichtung nach einem der Ansprüche 1 bis 25, **dadurch gekennzeichnet, daß** der Förderer (9) in zumindest einem Wandbereich ein Sieb aufweist mit einer als Trennschnecke ausgebildeten, dem Sieb zugeordneten Förderschnecke (9.1).

29. Vorrichtung nach Anspruch 28, **dadurch gekennzeichnet, daß** die als Trennschnecke ausgebildete Förderschnecke (9.1) einseitig gelagert und oberhalb der Trennschnecke ein Förderkanal (9.3) für nicht auszusiebendes Fördergut innerhalb des Förderers (9) vorgesehen ist, der in eine Austragöffnung (9.4) mündet.

30. Vorrichtung nach Anspruch 28 oder 29, **dadurch gekennzeichnet, daß** die Außenwandung des Förderers (9) bereichsweise als Lochmantel ausgebildet ist, der mit der Trennschnecke einen Reibboden bildet.

31. Vorrichtung nach einem der Ansprüche 28 bis 30, **dadurch gekennzeichnet, daß** der Förderer (9) zwei oder mehrere in Förderrichtung hintereinander angeordnete Verstellsiebe (9.5) aufweist mit jeweils zumindest zwei mit Sieblöchern versehenen Siebelementen, die zur Veränderung des Siebungsgrades gegeneinander verschiebbar sind und durch eine Spannvorrichtung aneinander gehalten werden.

## Claims

1. A device for conveying biomass materials into, for example, a fermenter (10, 11) with at least one conveying stage comprising, for example, a screw conveyor as a conveyor (5), with a size-reducer (8) associated with the conveying stage and comprising size-reduction tools, for example a mill, wherein the conveying stage comprises a further conveyor (9), by means of which biomass material can be conveyed so as to bypass the size-reducer and can be fed to the biomass material coming from the size-reducer (8), **characterised in that** the conveyor (5) associated with the size-reducer emerges into the size-reducer (8) in a region of a side wall and, via the conveyor (5), a forced introduction takes place into the size-reducer (8) via a pressure worm provided in the conveyor (5).

2. The device according to claim 1, **characterised in that** the two conveyors (5, 9) of the conveying stage are aligned parallel to one another at least in sections.

3. The device according to claim 1 or 2, **characterised in that** the two conveyors (5, 9) of the conveying stage are connected in series in the conveying direction of the biomass material.

4. The device according to any one of claims 1 to 3, **characterised in that** a bypass opening (5.3), which can be opened and closed by a closure element (5.2), is provided between the conveyors (5, 9) of the conveying stage.

5. The device according to any one of claims 1 to 4, **characterised in that** admixture material can be fed to one of the two conveyors (5, 9) via a supply opening.

6. The device according to any one of claims 1 to 5, **characterised in that** the conveying speed of the conveyors (5, 9) can be regulated as a function of adjustable operating parameters.

7. The device according to any one of claims 1 to 6, **characterised in that** the further conveyor (9) of the conveying stage is provided in the device in a shaft (20.1) beneath the size-reducer (1) and beneath a region of the first conveyor (5) of the conveying stage.

8. The device according to any one of claims 1 to 7, **characterised in that** the size-reducer comprises a sieve unit (21), which can be transferred from a shut-down position into an operating position associated with the size-reduction tools (13) and can be transferred back again.

9. The device according to any one of claims 1 to 8, **characterised in that** the sieve unit (16) comprises a sieve (21) which can undergo translational movements.

10. The device according to claim 9, **characterised in that** the sieve (21) comprises a curved sieve surface.

11. The device according to any one of claims 8 to 10, **characterised in that**, in its operating position, the sieve unit (16) bounds the size-reduction tools (13) in sections and surrounds the size-reducer (8) in sections downwards towards a shaft (20.1).

12. The device according to any one of claims 1 to 11, **characterised in that** the sieve unit (16) comprises a sieve carriage (22).

13. The device according to any one of claims 1 to 12, **characterised in that** the sieve carriage (22) can be moved along by guides (23).

14. The device according to claim 12 or 13, **characterised in that** the sieve (21) can be actuated by hydraulic or pneumatic cylinders (24).

15. The device according to any one of claims 1 to 14, **characterised in that** the sieve unit (16) comprises a sieve (21) which can be traversed into an opening provided in the housing of the size-reducer and can be transferred through this opening into its operating position and its shut-down position outside the housing.

16. The device according to any one of claims 1 to 15, **characterised in that** the sieve unit (16) comprises two sieve elements lying one upon the other, which each comprise a plurality of sieve holes, which form sieve openings depending on the relative position of the sieve elements to one another, and the relative position of the sieve elements with respect to one another can be changed.

17. The device according to any one of claims 1 to 16, **characterised in that** a moisture sensor is assigned to the sieve unit (16) and a motor-driven positioning unit for the transfer of the sieve unit (16) out of its shut-down position into its operating position and back can be actuated depending on the result of the moisture content of the biomass material ascertained by the moisture sensor.

18. The device according to any one of claims 1 to 17, **characterised in that** the size-reducer (8) is constituted as a size-reduction mill with beater bars (8.1) and associated impact strips (8.2).

19. The device according to claim 18, **characterised in that** the impact strips (8.2) are constituted so as to be adjustable.

20. The device according to claim 19, **characterised in that** the impact strips (8.2) have an eccentrically disposed swivel pin and are constituted so as to be swivellable.

21. The device according to any one of claims 1 to 20, **characterised in that** the size-reducer (8) comprises a cutting insert (30) associated with the size-reduction tools (13).

22. The device according to claim 21, **characterised in that** the cutting insert (30) comprises cutting blades (31) mounted in a swivellable manner.

23. The device according to claim 22, **characterised in that** the cutting blades (31) can each be held in a working position by means of a pressure spring (31.1) and, when acted upon by foreign bodies, are mounted with an individual evasion capability.

24. The device according to any one of claims 21 to 23, **characterised in that** the cutting insert (30) can be supported on a mobile blade cross-member (31.2) and can be transferred into an operating position and into a shut-down position by means of the blade cross-member (31.2) and an actuation device (35).

25. The device according to any one of claims 1 to 24, **characterised in that** the impact strips (8.2) are provided on an impact strip insert (36), which is linked in a swivellable manner to a housing of the size-reducer (8) and can be transferred into an operating position and into a shut-down position.

26. The device according to claim 25, **characterised in that** at least two impact strip inserts (36) are provided, whereof the one or the other can be partially transferred into the operating position.

27. The device according to any one of claims 1 to 26, **characterised in that** the device comprises an impact strip insert (36) on one side and a cutting tool insert on the other side, which can alternatively be transferred into the operating position.

28. The device according to any one of claims 1 to 25, **characterised in that** the conveyor (9) comprises a sieve in at least one wall region, with a screw conveyor (9.1) associated with the sieve and constituted as a separating screw.

29. The device according to claim 28, **characterised in that** the screw conveyor (9.1) constituted as a separating screw is supported on one side and, above the separating screw, a conveying channel (9.3) for conveyed material not to be screened out is provided inside the conveyor (9), said conveying channel emerging into a discharge opening (9.4).

30. The device according to claim 28 or 29, **characterised in that** the outer wall of the conveyor (9) is constituted in sections as a perforated shell, which forms a frictional base with the separating screw.

31. The device according to any one of claims 28 to 30, **characterised in that** the conveyor (9) comprises two or more adjustable sieves (9.5) disposed one behind the other in the conveying direction, with in each case at least two sieve elements, which are provided with sieve holes and which can be displaced with respect to one another in order to change the degree of sieving and are held to one another by a tensioning device.

## Revendications

1. Dispositif pour le transport de matériaux de biomasse dans, par exemple, un fermenteur (10, 11), avec, au minimum, une étape de transfert présentant par exemple un transporteur à vis sans fin en tant que convoyeur (5), avec, agencé dans l'étape de transfert, un broyeur (8) présentant des outils de broyage, par exemple, un moulin, l'étape de transfert présentant un convoyeur (9) supplémentaire par lequel le matériau de biomasse est transportable en évitant le broyeur et où le matériau de biomasse provenant du broyeur (8) peut être ajouté, **caractérisé en ce que** le convoyeur (5), agencé à côté du broyeur, débouche dans une zone de la paroi latérale du broyeur (8) et est abouté de force dans le broyeur (8) par le convoyeur (5) grâce à une vis de compression prévue dans le convoyeur (5).

2. Dispositif selon la revendication 1, **caractérisé en ce que** les deux convoyeurs (5, 9) de l'étape de transfert sont agencés parallèlement l'un à l'autre au moins dans certaines zones.

3. Dispositif selon la revendication 1 ou 2 **caractérisé en ce que** les deux convoyeurs (5, 9) de l'étape de transfert sont mis en circuit en série dans la direction de transport du matériau de biomasse.

4. Dispositif selon l'une des revendications 1 à 3 **caractérisé en ce qu'**une ouverture by-pass (5.3) est prévue entre les convoyeurs (5, 9) de l'étape de transfert, dont l'ouverture est libérée et fermée par un élément de fermeture (5.2).

5. Dispositif selon l'une des revendications 1 à 4, **caractérisé en ce que** l'un des deux convoyeurs (5, 9) est accessible par une ouverture d'alimentation pour de la matière additionnelle.

6. Dispositif selon l'une des revendications 1 à 5, **caractérisé en ce que** la vitesse de transport des convoyeurs (5, 9) est ajustable en fonction de paramètres de fonctionnement réglables.

7. Dispositif selon l'une des revendications 1 à 6, **caractérisé en ce que** le convoyeur supplémentaire (9) de l'étape de transfert est prévu être dans une cage (20.1) sous le broyeur (1) et en dessous de la zone du premier convoyeur (5) de l'étape de transfert dans le dispositif.

8. Dispositif selon l'une des revendications 1 à 7, **caractérisé en ce que** le broyeur présente une unité de tamisage (21) qui peut être transposée à partir d'une position hors service à une position de fonctionnement des outils de broyage (13) et peut être remise en place.

9. Dispositif selon l'une des revendications 1 à 8, **caractérisé en ce que** l'unité de tamisage (16) se présente sous la forme d'un tamis (21) mobile de façon translatoire.

10. Dispositif selon la revendication 9, **caractérisé en ce que** le tamis (21) présente une surface de tamisage courbe.

11. Dispositif selon l'une des revendications 8 à 10, **caractérisé en ce que** l'unité de tamisage 16), dans sa position de fonctionnement, limite partiellement les outils de broyage (13) et entoure partiellement le broyeur (8), vers le bas, d'une cage (20.1).

12. Dispositif selon l'une des revendications 1 à 11, **caractérisé en ce que** l'unité de tamisage (16) présente un coulisseau de tamis (22).

13. Dispositif selon l'une des revendications 1 à 12, **caractérisé en ce que** le coulisseau de tamis (22) peut se déplacer le long de guides (23).

14. Dispositif selon la revendication 12 ou 13, **caractérisé en ce que** le tamis (21) peut être mis en oeuvre par des cylindres hydrauliques ou pneumatiques (24).

15. Dispositif selon l'une des revendications 1 à 14, **caractérisé en ce que** l'unité de tamisage (16) présente un tamis (21) que l'on peut mettre en place par une ouverture prévue dans l'enceinte du broyeur et peut être disposé par cette ouverture dans sa position de fonctionnement et dans sa position hors service à l'extérieur de l'enceinte.

16. Dispositif selon l'une des revendications 1 à 15, **caractérisé en ce que** l'unité de tamisage (16) présente deux éléments de tamisage superposés, qui chaque fois présentent une multitude d'orifices qui forment des orifices de tamisage en fonction de la position relative des éléments de tamisage les uns par rapport aux autres et la position relative des éléments de tamisage entre eux est modulable.

17. Dispositif selon l'une des revendications 1 à 16, **caractérisé en ce que** l'unité de tamisage (16) est adjointe d'un capteur d'humidité et qu'une unité de commutation motorisée pour le passage de l'unité de tamisage (16) en position de fonctionnement à partir de sa position hors service, peut être mise en service en fonction du résultat en humidité du matériau de biomasse donné par le capteur d'humidité.

18. Dispositif selon l'une des revendications 1 à 17, **caractérisé en ce que** le broyeur (18) est sous la forme d'une installation de broyage avec des barres d'impact (8.1) et des absorbeurs de chocs (8.2) intégrés.

19. Dispositif selon la revendication 18, **caractérisé en ce que** les absorbeurs de chocs (8.2) sont conçus de manière réglable.

20. Dispositif selon la revendication 19 **caractérisé en ce que** les absorbeurs de chocs (8.2) possèdent un axe orientable agencé de façon excentrique et sont conçus pour pouvoir tourner.

21. Dispositif selon l'une des revendications 1 à 20, **caractérisé en ce qu'**un des outils de broyage (13) du broyeur (8) présente une plaquette de coupe (30) intégrée.

22. Dispositif selon la revendication 21, **caractérisé en ce que** la plaquette de coupe (30), présente des outils coupants (31) rangés qui peuvent tourner.

23. Dispositif selon la revendication 22, **caractérisé en ce que** les outils coupants (31) peuvent être chaque fois maintenus en position de fonctionnement grâce à un ressort de pression (31.1) et sont rangés,
pouvant être dégagés individuellement par l'impact d'un corps étranger.

24. Dispositif selon l'une des revendications 21 à 23 **caractérisé en ce que** la plaquette de coupe (30) peut s'appuyer sur un support transverse de couteaux (31.2) mobile et peut être actionnée à une position de fonctionnement et une position hors service par l'intermédiaire du support transverse de couteaux (31.2) et d'un dispositif de mise en marche (35).

25. Dispositif selon l'une des revendications 1 à 24 **caractérisé en ce que** les absorbeurs de chocs (8.2) sont prévus être dans un compartiment d'absorbeurs de chocs (36) qui peut tourner dans une enceinte accotée au broyeur (8) et qui peut être actionné à une position de fonctionnement et à une position hors service.

26. Dispositif selon la revendication 25 **caractérisé en ce qu'**au moins deux compartiments d'absorbeurs de chocs (36) sont prévus, parmi lesquels l'un ou l'autre peut être actionné partiellement en position de fonctionnement.

27. Dispositif selon l'une des revendications 1 à 26 **caractérisé en ce que** le dispositif présente sur un côté un compartiment pour les absorbeurs de chocs (36) et sur l'autre côté un compartiment pour les outils de coupe qui peuvent être mis au choix en position de fonctionnement.

28. Dispositif selon l'une des revendications 1 à 25 **caractérisé en ce que** le convoyeur (9) présente sur au moins l'une de ses parois un tamis avec un transporteur à vis (9.1) à côté du tamis et en forme de séparateur à vis.

29. Dispositif selon la revendication 28 **caractérisé en ce que** le transporteur à vis (9.1) en forme de séparateur à vis est conçu pour se situer d'un côté et qu'il y ait une canalisation de transport (9.3), au-dessus du séparateur à vis, pour de la matière à transporter non tamisée à l'intérieur du convoyeur (9), qui débouche dans un orifice de sortie (9.4).

30. Dispositif selon la revendication 28 ou 29 **caractérisé en ce que** la paroi externe du convoyeur (9) se présente partiellement sous la forme d'un revêtement troué qui forme avec le séparateur à vis une zone de frottement.

31. Dispositif selon l'une des revendications 28 à 30 **caractérisé en ce que** le convoyeur (9) présente deux ou plusieurs tamis réglables (9.5) alignés les uns derrière les autres dans la direction de transport avec chaque fois au moins deux éléments de tamisage pourvus d'orifices de tamisage qui peuvent être déplacés les uns par rapport aux autres pour la variation du degré de tamisage et qui sont maintenus ensemble par un dispositif de tension.
